# EUROPEAN PATENT SPECIFICATION

(11) **EP 3 211 578 B1**
(45) Date of publication and mention of the grant of the patent: **30.09.2020**
(21) Application number: 15851912.4
(22) Date of filing: 10.10.2015
(51) Int. Cl.: H04W 12/08, G06Q 20/40, G06F 21/32, G06F 21/62, H04L 29/06

(54) **VERIFICATION METHOD AND APPARATUS**
VERIFIZIERUNGSVERFAHREN UND -VORRICHTUNG
PROCÉDÉ ET APPAREIL DE VÉRIFICATION

(30) Priority: 20.10.2014 CN 201410559635
(43) Date of publication of application: 30.08.2017
(73) Proprietor: Alibaba Group Holding Limited, Grand Cayman (KY)
(72) Inventor: YANG, Ke, Hangzhou Zhejiang 311121 (CN)
(74) Representative: Branderhorst, Matthijs Pieter Arie
(86) International application number: PCT/CN2015/091688
(87) International publication number: WO 2016/062198

(56) References cited:
- WO-A1-03/001866
- CN-A- 103 870 220
- CN-A- 103 970 271
- CN-A- 104 102 867
- CN-A- 104 574 088
- CN-A- 104 850 827
- CN-A- 104 850 995
- US-A1- 2012 215 328
- US-A1- 2014 237 028
- GAO, YUAN ET AL.: 'Smartphone-based Wearable, Ambulatory Monitoring System' CHINESE MEDICAL EQUIPMENT vol. 31, no. 05, 31 May 2010, XP009502407

## Description

### Technical Field

The present application relates to the field of communication technologies, and in particular, to a verification method and apparatus.

### Background Art

With extensive development of the Internet technology, information security problems are particularly acute. Identity verification is generally needed when people use terminals for entertainment and shopping.

Verification methods at present generally include: password verification and environment verification. For example, when a user logs in or makes a payment, it is verified whether a password input by the user is a preset password, or whether a browser or APP (Application) used by the user has Trojans and viruses installed therein. However, this method starts verification only when an account of a user has a login or payment behavior. If a terminal of a user is lost or information of an account of the user is stolen, the security of the account of the user cannot be guaranteed.

Document US 2012/0215328 discloses systems, methods, and apparatus for monitoring motion or other activity and using the successful completion of a goal to unlock or lock functionality or features of an electronic device or applications running on a computing system.

Document EP 3121706 discloses a control method and a control system for a working mode of a mobile device.

### Summary

The invention provides a verification method and a verification apparatus as set out in the accompanying claims.

It can be seen that the present application may send to a server physiologic information, of a user, collected by a device bound by the user, such that the server may determine, according to the physiologic information, whether the user is currently in a non-operation state, and when the user is in a non-operation state, process, according to a preset rule, an operation request initiated by the user, thus enhancing the security of an account of the user, and providing real-time protection for the account of the user.

### Brief Description of the Drawings

FIG. 1 is a schematic flow chart of a verification method according to an embodiment of the present application;
FIG. 2 is a schematic flow chart of a verification method according to another embodiment of the present application;
FIG. 3 is a schematic flow chart of a verification method according to another embodiment of the present application;
FIG. 4 is a schematic structural diagram of a server according to an embodiment of the present application;
FIG. 5 is a schematic structural diagram of a verification apparatus applied to a server according to an embodiment of the present application;
FIG. 6 is a schematic structural diagram of a client according to an embodiment of the present application; and
FIG. 7 is a schematic structural diagram of a verification apparatus applied to a client according to an embodiment of the present application.

### Detailed Description

Exemplary embodiments will be described in detail herein, and examples thereof are shown in the accompanying drawings. In the following description involving the accompanying drawings, the same numerals in different accompanying drawings denote the same or similar elements, unless otherwise specified. Implementations described in the following exemplary embodiments do not represent all implementations consistent with the present application. In contrast, they are merely examples of apparatuses and methods consistent with some aspects of the present application as described in detail in the appended claims.

Terms used in the present application are merely used for describing specific embodiments, instead of limiting the present application. Singular forms "a(n)", "said", and "the" used in the present application and the appended claims are also intended to include plural forms, unless clearly specified in the context to have other meanings. It should be further understood that the term "and/or" used herein refers to and includes any or all possible combinations of one or more associated items that are listed.

It should be understood that, although terms such as "first", "second", and "third" may be used in the present application to describe various kinds of information, these kinds of information should not be limited to the terms. These terms are merely used to distinguish information of the same type from each other. For example, without departing from the scope of the present application, the first information may also be referred to as the second information, and similarly, the second information may also be referred to as the first information. Depending on the context, the word "if' used herein may be explained as "when...", "as...", or "in response to the determination".

With respect to the above problems, the present application provides a verification solution, which can effectively enhance the security of an account of a user.

The present application provides a verification method, and the method is applied to a server and a client or device terminal capable of interacting with the server, respectively.

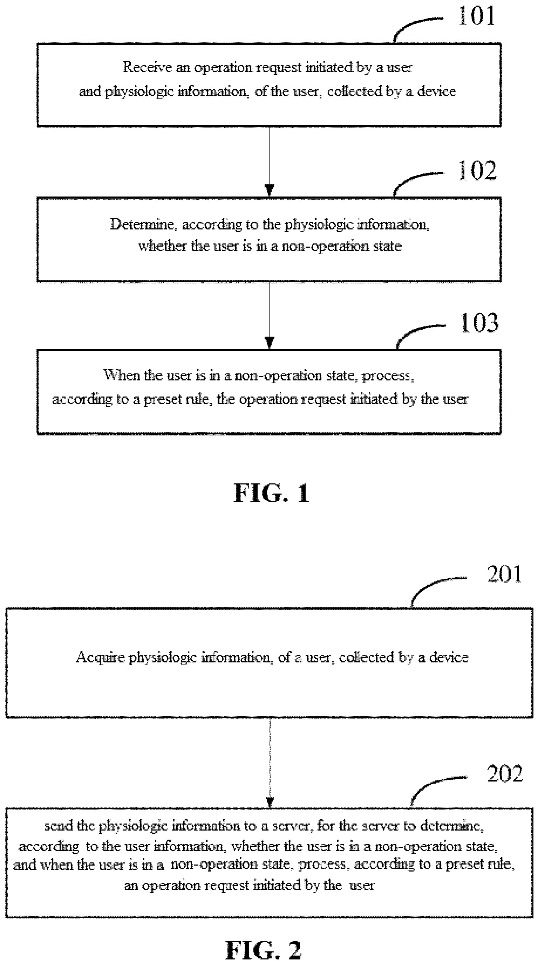

Referring to FIG. 1, the verification method applied to a server includes the following steps:
Step 101: An operation request initiated by a user and physiologic information, of the user, collected by a device are received.

In this embodiment, the user may bind an account of the user with the device, and the device includes a wearable device, for collecting the physiologic information of the user, and is capable of sending the physiologic information to the server.

The physiologic information may include: physiologic indexes such as heartbeat, body temperature, and pulse.

The operation request initiated by the user is an operation request of the user on the account of the user, such as login, modification information, and payment, which is not limited in the present application.

Step 102: It is determined, according to the physiologic information, whether the user is in a non-operation state.

Based on the step 101, after receiving the physiologic information, the server may determine, according to the physiologic information, whether the user is in a non-operation state. The non-operation state may be understood as a state where the user cannot use the application software, for example, the user can barely use some application software when sleeping or exercising, and therefore, the non-operation state may include: sleeping or exercising.

Step 103: When the user is in a non-operation state, the operation request initiated by the user is processed according to a preset rule.

In this embodiment, if the server determines, according to the physiologic information, that the user is currently in a non-operation state, the server may process, according to the preset rule, the operation request initiated by the user to enhance the security of the account of the user. For example, the server may add a verification manner for the account of the user, reject the operation request initiated by the user, or the like.

Referring to FIG. 2, the verification method applied to a client or a device terminal includes the following steps:
Step 201: Physiologic information, of a user, collected by a device is acquired.

In this embodiment, the device may include: a wearable device. The physiologic information may include: physiologic indexes, such as heartbeat, body temperature, pulse, etc., of the user.

Step 202: The physiologic information is sent to a server, for the server to determine, according to the user information, whether the user is in a non-operation state, and when the user is in a non-operation state, process, according to a preset rule, an operation request initiated by the user.

Based on the step 201, the user may install a client on the device, and the client may acquire the physiologic information, of the user, collected by the device, and send the physiologic information to the server. Definitely, a preset program or chip may be configured on the device in advance, for sending the physiologic information to the server after the device collects the physiologic information of the user. Specifically, in this step, the user information may be sent to the server periodically, and the sending period may be set by a developer, for example, 2 seconds, which is not specially limited in the present application.

It can be seen from the above description that the present application may send physiologic information, of a user, collected by a device bound by the user to a server, such that the server may determine, according to the physiologic information, whether the user is in a non-operation state, and when the user is in a non-operation state, process, according to a preset rule, an operation request initiated by the user, thus enhancing the security of an account of the user, and providing real-time protection for the account of the user.

A specific implementation of the present application is described below in detail with reference to embodiments.

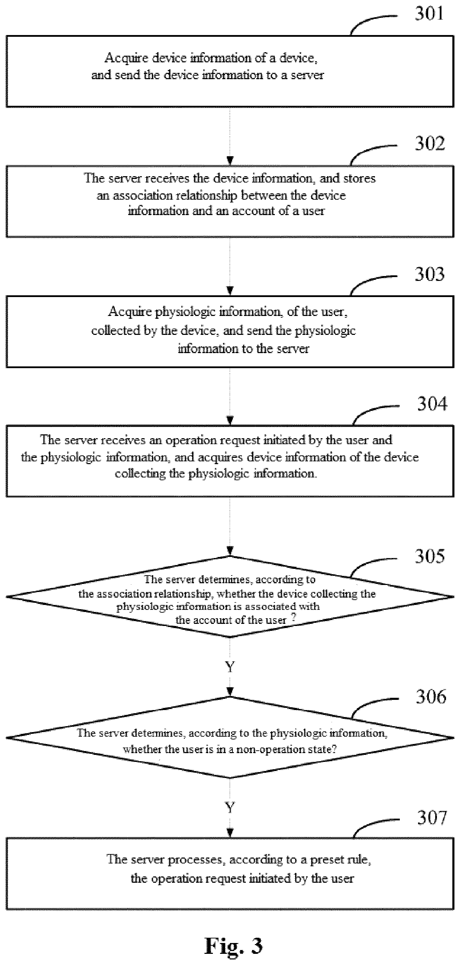

Referring to FIG. 3, a schematic flow chart of a verification method according to an embodiment of the present application is shown, and the method includes the following steps:
Step 301: Device information of a device is acquired, and the device information is sent to a server.

In this embodiment, a user may choose, according to his/her own requirements, whether to enable the device to participate in a security decision of an account of the user, and if the user chooses to enable the device to participate in the security decision of the account of the user, the user needs to bind the device with the account of the user.

Specifically, a preset program or chip may be configured on the device, for sending the device information to the server after the device is started. Also, a client may be installed on the device, and the client may acquire device information of the device, such as a device model, and a device MAC address, and then send the device information to the server. Further, the device may be bound through a client or browser loaded on another terminal. For example, the user may log in to the account of the user through a browser, and then manually input device information of the device that the user intends to bind. Illustrations are made by taking loading a client on the device as an example in the following.

In this embodiment, the device may include: a wearable device such as a smart band and a smart watch, which is not limited in the present application.

Step 302: The server receives the device information, and stores an association relationship between the device information and the account of the user.

Based on the step 301, after receiving the device information of the device, the server may bind the device with the account of the user by storing the association relationship between the device information and the account of the user.

The user may bind the same account of the user with multiple devices. For example, the user may bind the smart band and the smart watch with a user account A used by him/her, and the server stores association relationships between the user account A and the smart band as well as the smart watch. It should be noted that, although the user may bind multiple devices with the same account of the user, generally, the user can only select one device to participate in the security decision of the account of the user. For example, if the user first binds the smart band with the account A of the user, and then binds the smart watch with the account A of the user, a user prompt may be generated when the user performs binding, to prompt the user to select one of the smart band and the smart watch to participate in the security decision of the account of the user, and the server may store the user's selection, and set the state of the device selected by the user as an available state.

Step 303: Physiologic information, of the user, collected by the device is acquired, and the physiologic information is sent to the server.

In this embodiment, the client acquires the physiologic information, of the user, collected by the device, and the physiologic information may include: physiologic indexes, such as heartbeat, body temperature, and pulse, etc., of the user. Then, the physiologic information is sent to the server.

Specifically, in this step, the client may send the physiologic information to the server through a wireless network which the device accesses. If the device does not access a wireless network currently, the client may send the physiologic information to the server through a terminal that matches the device, which is not limited in the present application.

Step 304: The server receives an operation request initiated by the user and the physiologic information, and acquires device information of the device collecting the physiologic information.

Based on the step 303, after receiving the physiologic information sent by the client, the server acquires the device information of the device collecting the physiologic information. Specifically, when sending the physiologic information to the server, the client sends the device information of the device together to the server.

In this step, the server further receives the operation request initiated by the user on the account of the user, and the operation request includes: login, modification information, payment, and the like, which is not limited in the present application.

Step 305: The server determines, according to the association relationship, whether the device collecting the physiologic information is associated with the account of the user, and step 306 is performed if the device is associated with the account of the user. If the device is not associated with the account of the user, the procedure is ended.

In this embodiment, the server determines, according to the device information of the device bound with the account of the user stored in step 302, whether the device collecting the physiologic information is associated with the account of the user, and if the device collecting the physiologic information is associated with the account of the user, it indicates that the device collecting the physiologic information is legal, the physiologic information is available, and step 306 is performed. If the device collecting the physiologic information is not associated with the account of the user, it indicates that the device collecting the physiologic information is not legal, the physiologic information is unavailable, and the procedure is ended.

Further, if the user binds multiple devices with the same account of the user, after the server acquires the device information of the device collecting the physiologic information, if it is determined that the device is associated with the account of the user, it is determined whether the state of the device is an available state, and if the state of the device is an available state, the physiologic information is available, and step 306 is performed. If the state of the device is an unavailable state, the physiologic information is unavailable, and the procedure is ended. For example, the user binds the smart band and the smart watch with the account A of the user, assuming that the device used by the user currently is the smart band, and the bound smart watch is used by a family member, the state of the smart band may be set as an available state, and after the server receives the physiologic information, if the physiologic information is collected by the smart band, the physiologic information is available. If the physiologic information is collected by the smart watch, the physiologic information is unavailable.

Step 306: The server determines, according to the physiologic information, whether the user is in a non-operation state, and if the user is in a non-operation state, step 307 is performed. If the user is in an operation state, the procedure is ended.

Based on the step 305, after determining that the physiologic information is available, the server determines a current state of the user according to the physiologic information. Specifically, according to physiologic indexes, such as heartbeat, body temperature, and pulse, of the user, it may be determined whether the user is currently in a non-operation state, for example, sleeping or exercising. If the user is in a non-operation state currently, step 307 is performed. If the user is in an operation state, for example, the user is not sleeping nor exercising currently, the procedure is ended.

Step 307: The server processes, according to a preset rule, the operation request initiated by the user.

Based on the determination result of the step 306, if the user is in a non-operation state, the probability that the user uses an account of the user to make a login or payment behavior is extremely small. At this point, if the server receives the operation request initiated by the user, the probability that the operation request is initiated by the owner of the account of the user is extremely small, and the operation request may be processed according to the preset rule, to enhance the security of the account of the user. For example, at least one verification manner is added for an account of the user. The verification manner includes: one or more of two-dimensional code verification, short message verification code verification, and security question verification. Assuming that the original login verification manner of the account of the user is password verification, if the user is in the non-operation state, when receiving a login operation request for the account of the user, the server may add a short message verification code verification for the account of the user. In an extreme case, the operation request initiated by the user may be rejected when the user is in the non-operation state. Preferably, the operation request initiated by the user is rejected when multiple verifications fail. This is because, although the user is in the non-operation state, the user may lend his/her own account to another person for use, and in this step, the received operation request may be processed according to the user's pre-setting.

Optionally, in another implementation of the present application, if the server does not receive the physiologic information within a preset time, the server may send an alert to a management device preset by the user. The preset time may be set by a developer. Assuming that the client sends the user information to the server every 2 seconds, the preset time may be set as 10 seconds, that is, if the server does not receive the physiologic information within 10 seconds, the server may send an alert to a mobile phone used by the user through a mobile phone number reserved by the user.

Specifically, the server does not receive the physiologic information within the preset time, and this may be caused by a network failure or loss of the device bound by the user; at this point, the server sending an alert may remind the user to check the current situation. Further, the user may preset a processing strategy for the case that the server does not receive the physiologic information within the preset time, and the processing strategy may include: closing login and payment functions of the account of the user, that is, rejecting all operation requests initiated by the user, or the device does not participate in the security decision of the account of the user, or the like, which is not specially limited in the present application.

It can be seen from the above description that, the present application may send the physiologic information, of the user, collected by the device bound by the user to the server, such that the server determines, according to the physiologic information, whether the user is currently in a non-operation state, and when the user is in a non-operation state, processes, according to a preset rule, the operation request initiated by the user, thus enhancing the security of the account of the user, and providing real-time protection for the account of the user.

Corresponding to the embodiment of the verification method of the present application, the present application further provides a verification apparatus. The apparatus of the present application may be implemented through software, and may also be implemented by hardware or a combination of software and hardware. By using software implementation as an example, as an apparatus in a logic sense, the verification apparatus of the present application is formed by reading a corresponding computer program instruction in a non-volatile memory, by a processor of a device thereof, into a memory for running.

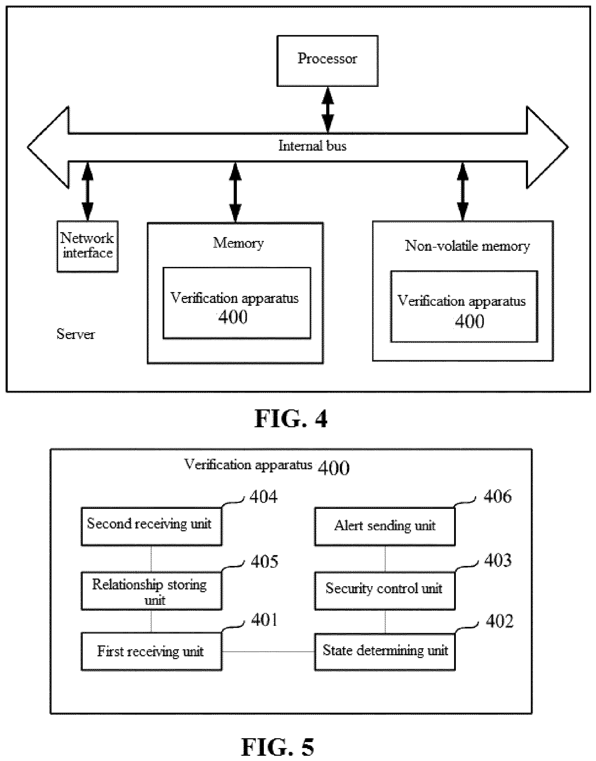

Referring to FIG. 4 and FIG. 5, the present application provides a verification apparatus 400, and the verification apparatus 400 is applied to a server, including: a first receiving unit 401, a state determining unit 402, a security control unit 403, a second receiving unit 404, a relationship storing unit 405, and an alert sending unit 406.

The first receiving unit 401 is configured to receive an operation request initiated by a user and physiologic information, of the user, collected by a device.

The state determining unit 402 is configured to determine, according to the physiologic information, whether the user is in a non-operation state.

The security control unit 403 is configured to, when the user is in a non-operation state, process, according to a preset rule, the operation request initiated by the user.

Further, the security control unit 403 is configured to specifically add at least one verification manner for an account of the user; or rejects the operation request initiated by the user.

The second receiving unit 404 is configured to receive device information of the device.

The relationship storing unit 405 is configured to store an association relationship between the device information and the account of the user.

The first receiving unit 401 is configured to specifically acquire the device information of the device collecting the physiologic information, after receiving the physiologic information, of the user, collected by the device.

The state determining unit 402 is configured to specifically determine, according to the association relationship, whether the device is associated with the account of the user, and determine, according to the physiologic information, whether the user is in a non-operation state if the device is associated with the account of the user.

The alert sending unit 406 is configured to send an alert to an associated device preset by the user if the physiologic information is not received within a preset time.

Further, the physiologic information includes: one or more of heartbeat, body temperature, and pulse; and the non-operation state includes: sleeping or exercising.

Further, the device is a wearable device.

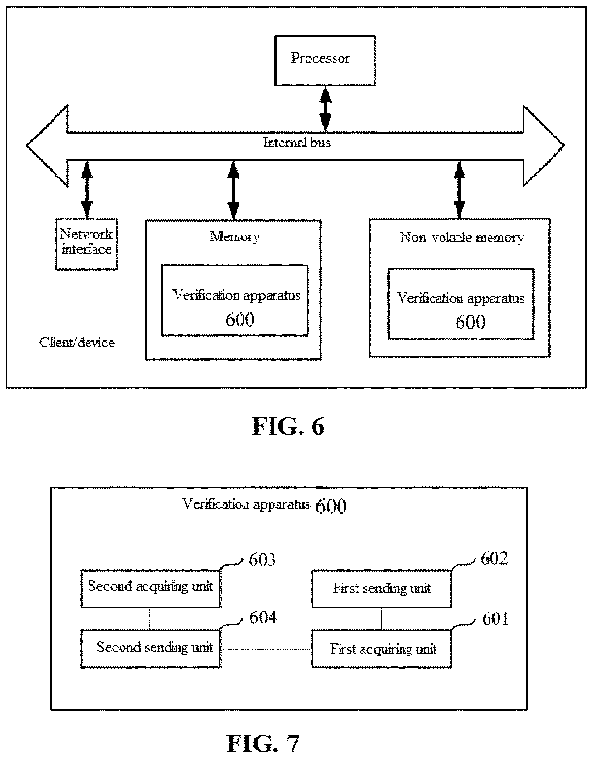

Referring to FIG. 6 and FIG. 7, the present application provides a verification apparatus 600, and the verification apparatus 600 is applied to a client, including: a first acquiring unit 601, a first sending unit 602, a second acquiring unit 603, and a second sending unit 604.

The first acquiring unit 601 is configured to acquire physiologic information, of a user, collected by a device.

The first sending unit 602 is configured to send the physiologic information to a server, for the server to determine, according to the physiologic information, whether the user is in a non-operation state, and when the user is in a non-operation state, process, according to a preset rule, an operation request initiated by the user.

The second acquiring unit 603 is configured to acquire device information of the device.

The second sending unit 604 is configured to send the device information to the server, for the server to store an association relationship between the device information and an account of the user, and determine, according to the association relationship, whether the device is associated with the account of the user.

Reference can be specifically made to the implementation processes of corresponding steps in the above method for implementation processes of functions and effects of the units in the above apparatus, which are not repeated herein.

The above descriptions are merely preferred embodiments of the present application, and are not intended to limit the present application. Any modification, equivalent replacement and improvement shall fall within the scope of the claims.

## Claims

1. A verification method, the method being performed by a verification apparatus and comprising:
defining a non-operation state of a user in which, if the user is in said non-operation state, it is unlikely that the user generates an operation request on an account of the user;
receiving (101, 304) the operation request on the account of the user and physiologic information of the user collected by a device used by the user;
determining whether the device is associated with the account of the user according to an association relationship between device information of the device and the account of the user;
in response to determining that the device is not associated with the account of the user, terminating processing of the operation request on the account of the user;
in response to determining that the device is associated with the account of the user;
determining (102, 306), according to the physiologic information, whether the user is currently in said non-operation state; and
when the user is determined to be in said non-operation state, processing (103, 307), according to a preset rule, the operation request on the account of the user, such that real-time protection of the account of the user is provided.

2. The method according to claim 1, wherein
the processing, according to the preset rule, the operation request initiated by the user comprises:
adding at least one verification manner for the account of the user; or
rejecting the operation request on the account of the user.

3. The method according to claim 1, wherein the method further comprises:
receiving the device information of the device;
storing the association relationship between the device information and the account of the user; and
wherein determining whether the device is associated with the account of the user according to an association relationship between device information of the device and the account of the user comprises:
acquiring the device information of the device collecting the physiologic information, after the physiologic information, of the user, collected by the device is received;
determining, according to the association relationship, whether the device collecting the physiologic information is associated with the account of the user.

4. The method according to claim 1, wherein the method further comprises:
sending an alert to an associated device preset by the user if the physiologic information is not received within a preset time.

5. The method according to claim 1, wherein
the physiologic information comprises: one or more of heartbeat, body temperature, and pulse; and
the non-operation state comprises: sleeping or exercising.

6. A verification apparatus, comprising:
a first receiving unit (401), configured to receive an operation request on an account of a user and physiologic information of the user collected by a device used by the user;
a state determining unit (402), configured to determine whether the device is associated with the account of the user according to an association relationship between device information of the device and the account of the user, and in response to determining that the device is associated with the account of the user, determine according to the physiologic information, whether the user is in a non-operation state, wherein the user is in said non-operation state if it is unlikely that the user generates the operation request on the account of the user; and
a security control unit (403), configured to process, when the user is determined to be in the non-operation state, according to a preset rule, the operation request on the account of the user, such that real-time protection of the account of the user is provided.

7. The apparatus according to claim 6, wherein
the security control unit (403) is configured to specifically add at least one verification manner for the account of the user; or reject the operation request on the account of the user.

8. The apparatus according to claim 6, wherein the apparatus further comprises:
a second receiving unit (404), configured to receive the device information of the device;
a relationship storing unit (405), configured to store the association relationship between the device information and the account of the user;
the first receiving unit (401), after receiving the physiologic information, of the user, collected by the device, configured to specifically acquire the device information of the device collecting the physiologic information; and
the state determining unit (402), configured to specifically determine, according to the association relationship, whether the device is associated with the account of the user.

9. The apparatus according to claim 6, wherein the apparatus further comprises:
an alert sending unit (406), configured to send an alert to an associated device preset by the user if the physiologic information is not received within a preset time.

10. The apparatus according to claim 6, wherein
the physiologic information comprises: one or more of heartbeat, body temperature, and pulse; and
the non-operation state comprises: sleeping or exercising.

11. The apparatus according to claim 6 or the method according to claim 1, wherein the device is a wearable device.

## Patentansprüche

1. Überprüfungsverfahren, wobei das Verfahren durch eine Überprüfungsgerät durchgeführt wird und Folgendes beinhaltet:
Definieren eines Nichtbetriebszustands eines Benutzers bei welchem, wenn der Benutzer in dem Nichtbetriebszustand ist, es unwahrscheinlich ist, dass der Benutzer eine Betriebsanfrage auf einem Account des Benutzers erzeugt;
Empfangen (101, 304) der Betriebsanfrage auf dem Account des Benutzers und von physiologischen Informationen des Benutzers, welche durch eine durch den Benutzer verwendete Vorrichtung gesammelt werden;
Bestimmen, ob die Vorrichtung dem Account des Benutzers gemäß einer Zuordnungsbeziehung zwischen Vorrichtungsinformationen der Vorrichtung und dem Account des Benutzers zugeordnet ist;
in Reaktion auf das Bestimmen, dass die Vorrichtung dem Account des Benutzers nicht zugeordnet ist, Beenden der Bearbeitung der Betriebsanfrage auf dem Account des Benutzers;
in Reaktion auf das Bestimmen, dass die Vorrichtung dem Account des Benutzers zugeordnet ist;
Bestimmen (102, 306), gemäß der physiologischen Informationen, ob der Benutzer augenblicklich in dem Nichtbetriebszustand ist; und
wenn bestimmt wird, dass der Benutzer in dem Nichtbetriebszustand ist, Bearbeiten (103, 307), gemäß einer vorgegebenen Regel, der Betriebsanfrage auf dem Account des Benutzers in einer Weise, dass Echtzeitschutz des Accounts des Benutzers bereitgestellt wird.

2. Verfahren nach Anspruch 1, wobei
die Bearbeitung, gemäß der vorgegebenen Regel, der Betriebsanfrage, welche durch den Benutzer initiiert wird, Folgendes beinhaltet:
Hinzufügen mindestens einer Überprüfungsart für den Account des Benutzers; oder
Zurückweisen der Betriebsanfrage auf dem Account des Benutzers.

3. Verfahren nach Anspruch 1, wobei das Verfahren zudem Folgendes beinhaltet:
Empfangen der Vorrichtungsinformationen der Vorrichtung;
Speichern der Zuordnungsbeziehung zwischen den Verrichtungsinformationen und dem Account des Benutzers; und
wobei Bestimmen, ob die Vorrichtung dem Account des Benutzers gemäß einer Zuordnungsbeziehung zwischen Vorrichtungsinformationen der Vorrichtung und dem Account des Benutzers zugeordnet ist, Folgendes beinhaltet:
Erfassen der Vorrichtungsinformationen der Vorrichtung, welche die physiologischen Informationen sammelt, nachdem die physiologischen Informationen des Benutzers, welche durch die Vorrichtung gesammelt wurden, empfangen wurden;
Bestimmen, gemäß der Zuordnungsbeziehung, ob die Vorrichtung, welche die physiologischen Informationen sammelt, dem Account des Benutzers zugeordnet ist.

4. Verfahren nach Anspruch 1, wobei das Verfahren zudem Folgendes beinhaltet:
Senden eines Alarms an eine durch den Benutzer vorgegebene zugeordnete Vorrichtung, wenn die physiologischen Informationen nicht innerhalb einer vorgegebenen Zeit empfangen werden.

5. Verfahren nach Anspruch 1, wobei
die physiologischen Informationen Folgendes beinhalten: eines oder mehrere von Herzschlag, Körpertemperatur und Puls; und
der Nichtbetriebszustand Folgendes beinhaltet: Schlafen oder Sporttreiben.

6. Überprüfungsgerät, Folgendes beinhaltend:
eine erste Empfangseinheit (401), konfiguriert zum Empfangen einer Betriebsanfrage auf einem Account eines Benutzers und von physiologischen Informationen des Benutzers, welche durch eine durch den Benutzer verwendete Vorrichtung gesammelt werden;
eine Zustandsbestimmungseinheit (402), konfiguriert zum Bestimmen, ob die Vorrichtung dem Account des Benutzers gemäß einer Zuordnungsbeziehung zwischen Vorrichtungsinformationen der Vorrichtung und dem Account des Benutzers zugeordnet ist, und, in Reaktion auf das Bestimmen, dass die Vorrichtung dem Account des Benutzers zugeordnet ist, Bestimmen, gemäß der physiologischen Informationen, ob der Benutzer in einem Nichtbetriebszustand ist, wobei der Benutzer in dem Nichtbetriebszustand ist, wenn es unwahrscheinlich ist, dass der Benutzer die Betriebsanfrage auf dem Account des Benutzers erzeugt; und
eine Sicherheitssteuerungseinheit (403), konfiguriert zum Bearbeiten, wenn bestimmt wurde, dass der Benutzer in dem Nichtbetriebszustand ist, gemäß einer vorgegebenen Regel der Betriebsanfrage auf dem Account des Benutzers in einer Weise, dass Echtzeitschutz des Accounts des Benutzers bereitgestellt wird.

7. Verfahren nach Anspruch 6, wobei
die Sicherheitssteuerungseinheit (403) dazu konfiguriert ist, spezifisch mindestens eine Überprüfungsart für den Account des Benutzers hinzuzufügen; oder die Betriebsanfrage auf dem Account des Benutzers zurückzuweisen.

8. Gerät nach Anspruch 6, bei welchem das Gerät zudem Folgendes beinhaltet:
eine zweite Empfangseinheit (404), konfiguriert zum Empfangen der Vorrichtungsinformationen der Vorrichtung;
eine Beziehungsspeichereinheit (405), konfiguriert zum Speichern der Zuordnungsbeziehung zwischen den Vorrichtungsinformationen und dem Account des Benutzers;
wobei die erste Empfangseinheit (401), nach dem Empfangen der physiologischen Informationen des Benutzers, welche durch die Vorrichtung gesammelt werden, konfiguriert ist, um spezifisch die Vorrichtungsinformationen der die physiologischen Informationen sammelnden Vorrichtung zu erfassen; und
wobei die Zustandsbestimmungseinheit (402) konfiguriert ist, um spezifisch gemäß der Zuordnungsbeziehung zu bestimmen, ob die Vorrichtung dem Account des Benutzers zugeordnet ist.

9. Gerät nach Anspruch 6, wobei das Gerät zudem Folgendes beinhaltet:
eine Alarmsendeeinheit (406), konfiguriert zum Senden eines Alarms an eine durch den Benutzer vorgegebene zugeordnete Vorrichtung, wenn die physiologischen Informationen nicht innerhalb einer vorgegebenen Zeit empfangen werden.

10. Verfahren nach Anspruch 6, wobei
die physiologischen Informationen Folgendes beinhalten: eines oder mehrere von Herzschlag, Körpertemperatur und Puls; und
der Nichtbetriebszustand Folgendes beinhaltet: Schlafen oder Sporttreiben.

11. Gerät nach Anspruch 6 oder Verfahren nach Anspruch 1, wobei die Vorrichtung eine tragbare Vorrichtung ist.

## Revendications

1. Procédé de vérification, le procédé étant réalisé par un appareil de vérification et comprenant :
la définition d'un état de non opération d'un utilisateur dans lequel, si l'utilisateur est dans ledit état de non opération, il est peu vraisemblable que l'utilisateur génère une requête d'opération sur un compte de l'utilisateur ;
la réception (101, 304) de la requête d'opération sur le compte de l'utilisateur et d'informations physiologiques de l'utilisateur qui sont collectées par un dispositif utilisé par l'utilisateur ;
la détermination de si le dispositif est ou n'est pas associé au compte de l'utilisateur en fonction d'une relation d'association entre des informations de dispositif du dispositif et le compte de l'utilisateur ;
en réponse à la détermination du fait que le dispositif n'est pas associé au compte de l'utilisateur, l'arrêt du traitement de la requête d'opération sur le compte de l'utilisateur ;
en réponse à la détermination du fait que le dispositif est associé au compte de l'utilisateur :
la détermination (102, 306), en fonction des informations physiologiques, de si l'utilisateur est ou n'est pas présentement dans ledit état de non opération ; et
lorsque l'utilisateur est déterminé comme étant dans ledit état de non opération, le traitement (103, 307), en fonction d'une règle prédéfinie, de la requête d'opération sur le compte de l'utilisateur, de telle sorte qu'une protection en temps réel du compte de l'utilisateur soit assurée.

2. Procédé selon la revendication 1, dans lequel :
le traitement, en fonction de la règle prédéfinie, de la requête d'opération qui est initiée par l'utilisateur comprend :
l'ajout d'au moins un mode de vérification pour le compte de l'utilisateur ; ou
le rejet de la requête d'opération sur le compte de l'utilisateur.

3. Procédé selon la revendication 1, dans lequel le procédé comprend en outre :
la réception des informations de dispositif du dispositif ;
le stockage de la relation d'association entre les informations de dispositif et le compte de l'utilisateur ; et
dans lequel la détermination de si le dispositif est ou n'est pas associé au compte de l'utilisateur en fonction d'une relation d'association entre des informations de dispositif du dispositif et le compte de l'utilisateur comprend :
l'acquisition des informations de dispositif du dispositif qui collecte les informations physiologiques, après que les informations physiologiques de l'utilisateur, qui sont collectées par le dispositif, sont reçues ;
la détermination, en fonction de la relation d'association, de si le dispositif qui collecte les informations physiologiques est ou n'est pas associé au compte de l'utilisateur.

4. Procédé selon la revendication 1, dans lequel le procédé comprend en outre :
l'envoi d'une alerte à un dispositif associé qui est prédéfini par l'utilisateur si les informations physiologiques ne sont pas reçues à l'intérieur d'un temps prédéfini.

5. Procédé selon la revendication 1, dans lequel :
les informations physiologiques comprennent : un ou plusieurs parmi le battement cardiaque, la température du corps et le pouls ; et
l'état de non opération comprend : le sommeil ou une activité sportive.

6. Appareil de vérification, comprenant :
une première unité de réception (401), qui est configurée pour recevoir une requête d'opération sur un compte d'un utilisateur et des informations physiologiques de l'utilisateur qui sont collectées par un dispositif utilisé par l'utilisateur ;
une unité de détermination d'état (402), qui est configurée pour déterminer si le dispositif est ou n'est pas associé au compte de l'utilisateur en fonction d'une relation d'association entre des informations de dispositif du dispositif et le compte de l'utilisateur, et, en réponse à la détermination du fait que le dispositif est associé au compte de l'utilisateur, pour déterminer, en fonction des informations physiologiques, si l'utilisateur est ou n'est pas dans un état de non opération, dans lequel l'utilisateur est dans ledit état de non opération s'il est peu vraisemblable que l'utilisateur génère la requête d'opération sur le compte de l'utilisateur ; et
une unité de commande de sécurité (403), qui est configurée pour traiter, lorsque l'utilisateur est déterminé comme étant dans l'état de non opération, en fonction d'une règle prédéfinie, la requête d'opération sur le compte de l'utilisateur, de telle sorte qu'une protection en temps réel du compte de l'utilisateur soit assurée.

7. Appareil selon la revendication 6, dans lequel :
l'unité de commande de sécurité (403) est configurée pour ajouter de façon spécifique au moins un mode de vérification pour le compte de l'utilisateur ; ou pour rejeter la requête d'opération sur le compte de l'utilisateur.

8. Appareil selon la revendication 6, dans lequel l'appareil comprend en outre :
une seconde unité de réception (404), qui est configurée pour recevoir les informations de dispositif du dispositif ;
une unité de stockage de relation (405), qui est configurée pour stocker la relation d'association entre les informations de dispositif et le compte de l'utilisateur ;
la première unité de réception (401), après la réception des informations physiologiques de l'utilisateur qui sont collectées par le dispositif, étant configurée pour acquérir de façon spécifique les informations de dispositif du dispositif qui collecte les informations physiologiques ; et
l'unité de détermination d'état (402), étant configurée pour déterminer de façon spécifique, en fonction de la relation d'association, si le dispositif est ou n'est pas associé au compte de l'utilisateur.

9. Appareil selon la revendication 6, dans lequel l'appareil comprend en outre :
une unité d'envoi d'alerte (406), qui est configurée pour envoyer une alerte à un dispositif associé qui est prédéfini par l'utilisateur si les informations physiologiques ne sont pas reçues à l'intérieur d'un temps prédéfini.

10. Appareil selon la revendication 6, dans lequel :
les informations physiologiques comprennent : un ou plusieurs parmi le battement cardiaque, la température du corps et le pouls ; et
l'état de non opération comprend : le sommeil ou une activité sportive.

11. Appareil selon la revendication 6 ou procédé selon la revendication 1, dans lequel le dispositif est un dispositif portatif.
